# EUROPEAN PATENT SPECIFICATION

(11) **EP 2 023 875 B1**
(45) Date of publication and mention of the grant of the patent: **12.02.2014**
(21) Application number: 07735638.4
(22) Date of filing: 24.04.2007
(51) Int. Cl.: A61F 13/551, A61F 13/15, A61F 15/00

(54) **ARRAY OF DISPOSABLE ABSORBENT ARTICLES HAVING TYPES OF DISCRETION**
ANORDNUNG VON SAUGFÄHIGEN EINWEG-ARTIKELN MIT UNTERSCHEIDUNGSARTEN
ENSEMBLE D'ARTICLES ABSORBANTS JETABLES ASSURANT DIFFERENTS TYPES DE DISCRETION

(30) Priority: 28.04.2006 US 413451
(43) Date of publication of application: 18.02.2009
(73) Proprietor: KIMBERLY-CLARK WORLDWIDE, INC., Neenah, WI 54956 (US)
(72) Inventor: HENDREN, Cynthia H., Winneconne, Wisconsin 54986 (US); SCHERMERHORN, James R., Neenah, Wisconsin 54956 (US); MULLEN, Erica L., Oshkosh, Wisconsin 54902 (US); FRANKE, Mark S., Neenah, Wisconsin 54956 (US); FITTON, Matthew T., Appleton, Wisconsin 54915 (US); MORTELL, Heather, S., Neenah, Wisconsin 54956 (US)
(74) Representative: Mabey, Katherine Frances
(86) International application number: PCT/IB2007/051514
(87) International publication number: WO 2007/125482

(56) References cited:
- WO-A-95/18589
- WO-A-02/096331
- US-A1- 2002 072 723
- US-A1- 2004 116 881

## Description

### BACKGROUND OF THE INVENTION

The present invention relates to absorbent articles, and more particularly to an array of disposable absorbent articles worn about the lower torso and having different types of discretion.

For some persons it is necessary to wear disposable absorbent garments about the lower torso to absorb and contain bodily exudates. For instance, some persons have or develop physical or mental problems where they become unable to adequately control the bowel or bladder and are considered incontinent. This may happen all of the time, or under certain situations, e.g. sleep, exercise, stress etc. For instance, sometimes young persons who are otherwise toilet-trained do not wake at night when their bladder needs to be voided. These persons then accidentally wet their beds until they physically and/or mentally mature enough to prevent the bedwetting incidents. For all such persons, regardless of age, incontinence can prove to be at times embarrassing, and lead to distress in social situations where their problem may be discovered by others.

US 2002/0072723 discloses an array of disposable absorbent article configurations. WO02/096331 discloses a package of disposable absorbent articles.

In an effort to assist those persons suffering from incontinence, very effective disposable absorbent garments have been developed that effectively contain bodily exudates. However, such protective undergarments may look like diapers or training pants used by babies and small children. For some persons this causes a loss of dignity, e.g. older children, and may lead to the avoidance of any social situation where others may accidentally see them in their protective undergarment. For others, it may lead to an avoidance of using protective undergarments.

### SUMMARY OF THE INVENTION

The present invention provides an array of disposable absorbent articles in accordance with claim 1.

In response to the discussed difficulties and problems encountered in the prior art, a new method of concurrently providing a series of types of garments with different types of discretion is provided. The array of disposable absorbent articles is adapted for a user in a particular life stage. The array is manufactured by or for the same business entity.

The array of disposable absorbent articles includes a first shell-covered absorbent garment comprising a first shell and an first absorbent member; and a second shell-covered garment comprising a second shell and an second absorbent member; and a non-covered absorbent garment.

Numerous features and advantages of the present invention will appear from the following description. In the description, reference is made to the accompanying drawings which illustrate desired embodiments of the invention. Such embodiments do not represent the full scope of the invention. Reference should, therefore, be made to the claims herein for interpreting the full scope of the invention.

### BRIEF DESCRIPTION OF THE DRAWINGS

Figure 1 is a rear elevational view of an array, the array having a first embodiment of a shell-covered garment and a non-covered garment;
Figure 2 is a front elevational view of a second embodiment of a shell-covered garment within the array of the present invention; where the shell has a skirt configuration;
Figure 3 is a front perspective view of a third embodiment of a shell-covered garment;
Figure 4 is a side perspective view of the garment shown in Figure 3, with a side opened for easy view of the garment interior;
Figure 5 is a plan view of one embodiment of the absorbent assembly used in the shell-covered garment of Figure 3;
Figure 6 is a plan view of one embodiment of the non-covered garment seen in Figure 1, shown in a disassembled, spread-out state;
Figure 7 is a front perspective view of the non-covered garment of Figure 6;

Corresponding parts are indicated by corresponding reference numbers throughout the drawings.

### DETAILED DESCRIPTION OF THE PREFERRED EMBODIMENT

The present invention is applicable to many different types of disposable absorbent articles that are fitted about the lower torso, including training pants, incontinence garments, enuresis garments, disposable underwear, and the like. It is especially (albeit not exclusively) suited to pant-like garments used for enuresis or incontinence needs.

Various terms used herein are defined as follows:
"Disposable" is a term used to refer to articles which are designed to be discarded after a limited use rather than being laundered or otherwise restored for reuse. For ease of explanation, the description hereafter will be in terms of an array of absorbent products directed to users suffering from enuresis-type incontinence. However, this is not meant to limit the invention to products directed toward this type of incontinence.

"Semi-private use" is a term used to indicate how a garment of the present invention will be used. If the garment may be seen by others when worn or stored, for instance at a slumber party, it may be considered a semi-private use of the garment.

"Private use" is another term used to indicate how a garment of the present invention will be used. If there are no others that may see the garment when worn or stored (with the exception of close family members or a person conducting a medical examination) it is considered a private use of the garment.

"Types of discretion" is used to refer to garment features that affect the ability of others to reasonably detect that a user is wearing a garment for reasons of incontinence. The primary garment features that affect one's ability to visually detect such garments based on its outward appearance when worn include: looseness of fit, percent of surface area covering the body, and opacity.

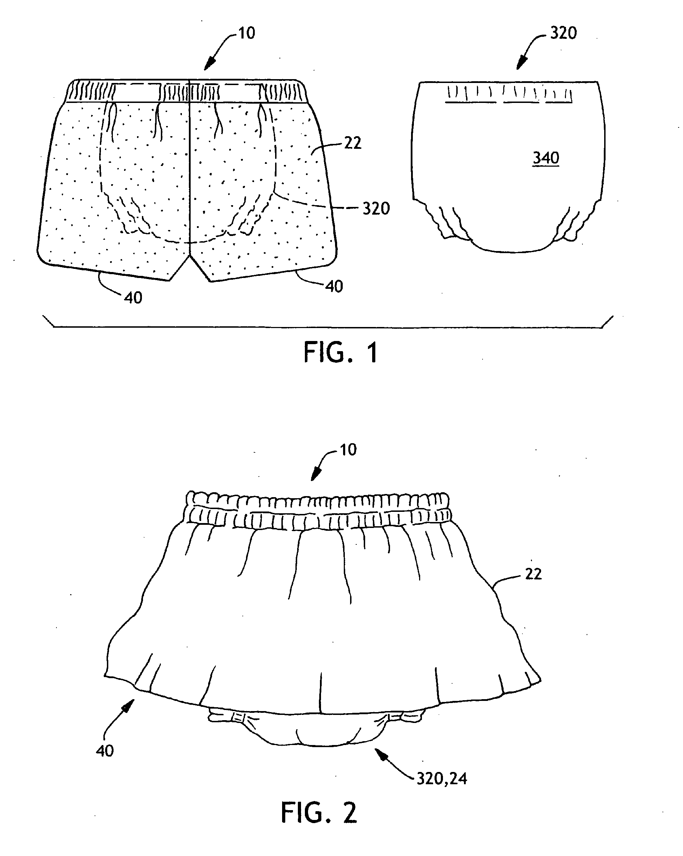

"Shell covered" is a term used to describe a garment such as a diaper, training pant, or other absorbent member that has a loose fitting outer cover or shell attached thereto, for example, at the waist. For instance, a boxer-short style shell 22 may cover a pull-on pant 320, and be attached to the pant at least partially at the waist (see Fig. 1). Several patents and patent applications disclose shell-covered garments; US2005/0131377 to Franke et al.; US Patent No. 6,168,585 to Cesco-Cancian; US Patent No. 6,115,847 and 6,009,558 and 5,876,394 to Rosch et al.; US Patent No. 6, 192,521 and 5,915,536 to Alberts et al.; EP 1329166 and EP 0955975 to Rosch et al.; US Serial No. 10/737101

(US20050131382); US. Serial No. 10/735978 (US20050131377); US Serial No. 10/736069 (US20050131381); US Serial No. 10/736443 (US20050125879). Each of these references is commonly owned by the assignee of the present invention.

"Non-covered" is a term used to refer to a diaper or pant-type garment that does not include an outer shell 22 (See Fig. 1).

"Boxer brief" is a term used to describe a garment such as a diaper, training pant, or other absorbent member that has an outer cover with extended or hanging legs. This outer cover is intended to be form fitting (close fitting) and may be constructed from a stretch material. The absorbent member may be attached directly to the outer cover either fully or partially, such as at the waist. See US Publication No. 2005/0256473.

"Life stage" is used herein to refer to where a user of the present invention is at in his or her life. Different than an array of absorbent articles designed for the stages of development experienced by a child going from diapers to training pants, and then to underwear, the users of the garments of the present invention may wear any appropriately-sized garments of the array at any age. Of course, users may eventually determine that there is no need to wear any of the garments in the array if the reason for which the garments are used ceases to exist; for example, the user no longer experiences incontinence.

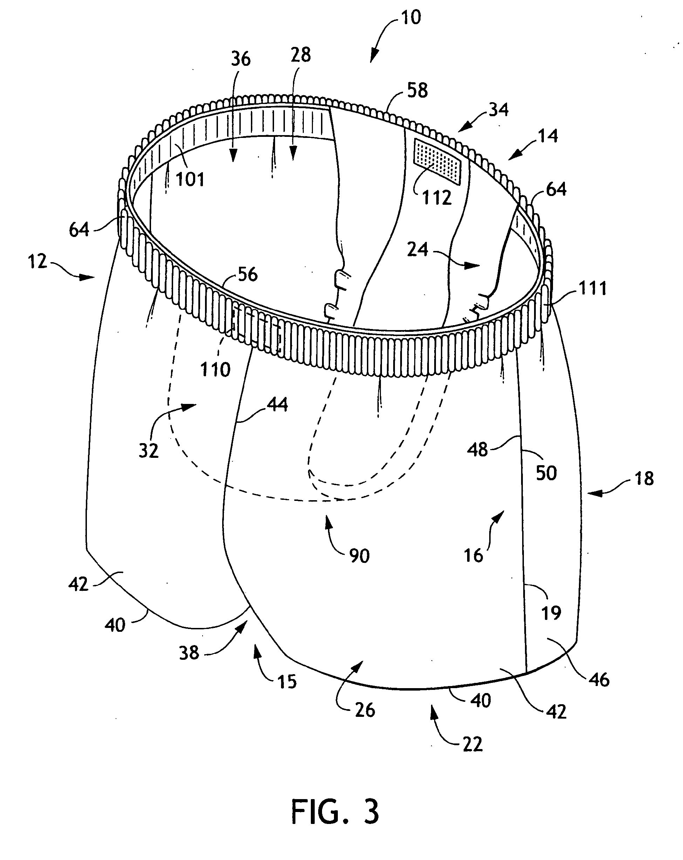

A "shell" is a relatively loose member which may be partially attached to an absorbent member or absorbent pant, typically at the waist region of the shell and the absorbent member or absorbent pant. Because the shell is loose, the absorbent member or pant underneath the shell is not readily apparent. The shell may be configured as a boxer short (see Fig. 3), a skirt (See Fig. 2), trousers (a boxer short with longer leg portions - not shown), bloomers (a boxer short with leg portions that are gathered at the edges to hug the leg or thigh of the user - not shown), or a skort (a boxer short with a skirt panel hanging over the front and or back of the garment - not shown).

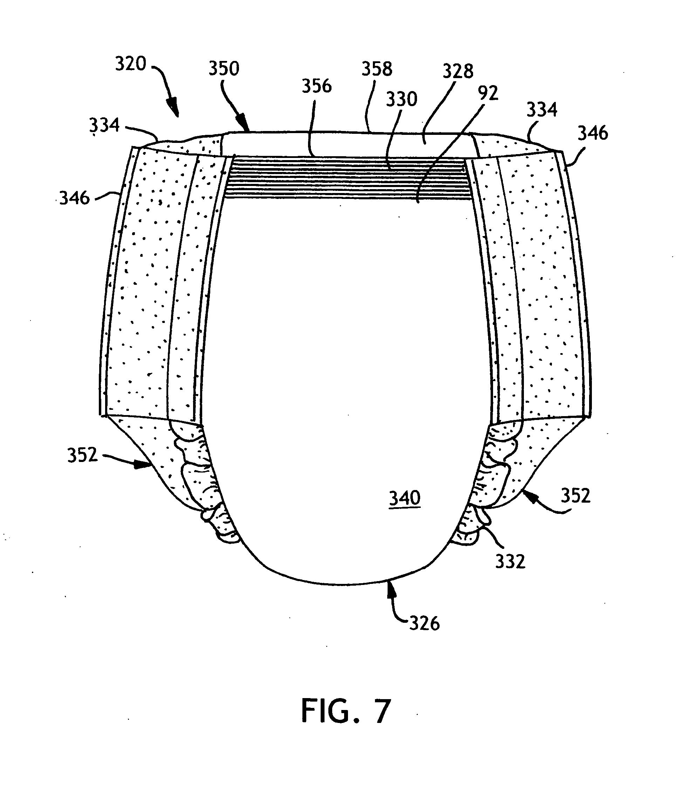

An array of the present invention includes a shell-covered absorbent garment 10 (Fig. 3) and at least one non-covered absorbent garment 320 (Fig. 7). In accordance with the invention the array also includes a second shell-covered absorbent garment. Each garment is manufactured by or for the same business entity and target the same user group. Each garment may offer a different type of discretion for the user; that is, one garment is easier to conceal from other persons in certain situations as described herein. The following garments are designed for use by a person at a single life stage.

Generally, a user of the array of the present invention experiences difficulty controlling his or her bowel or bladder habits, either all of the time, or at certain times of day or in certain situations. Since users suffer from incontinence, which may result from medical conditions such as hormonal imbalance, stroke, senility, and paralysis, or may be experienced during normal growth and development such as process of toilet training in children, it is anticipated that the user of the array of the present invention may be as young as about 3 years old; there is no limit as to how old the user might be.

The typical user of the present invention will have a desire or need to conceal his or her incontinence. Some users may want to have a more discreet garment for traveling outside the home, but may be willing to use any type of product for use in the privacy of his or her home. For example, a young person that experiences enuresis may want to participate in social situations such as sleeping away from home, e.g. at a friend or relatives home. For this purpose, this user may desire a garment that remains discreet, especially by the way the garment looks when worn by a user. This user may or may not require the same type of garment at home.

There is more than one style or type of disposable absorbent garment in the array of the present invention. "Type" as in "garment type" refers to a garment style or design that differs from another garment style or design, wherein the style or design may correlate to a functional aspect or appearance aspect that is included in one garment, and is excluded or is significantly different in another garment. There are several styles or designs of garments in the array of the present invention; including, a shell-covered garment and a non-covered garment as described herein. The array also includes a second shell-covered garment.

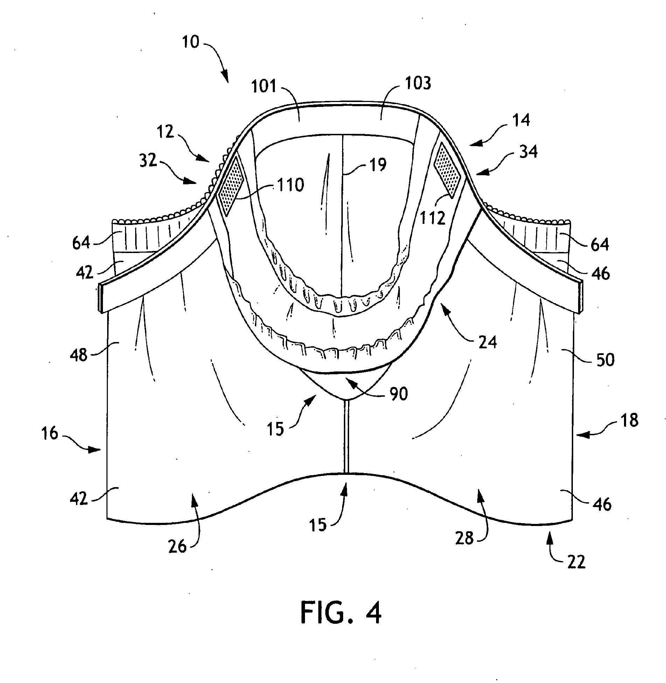

Referring now to the drawings and in particular to Figs. 3 and 4, an absorbent garment according to one embodiment of the present invention is indicated in its entirety by the reference numeral 10. In this embodiment, an absorbent assembly 24 may be permanently attached to a boxer-short style garment shell 22 so that the entire absorbent garment 10 is disposable. Alternatively, the absorbent assembly may be removable, adjustable, or replaceable within the shell 22. The absorbent garment 10 is configured to be worn on a wearer's waist and generally has a front waist region, indicated generally at 12, a back waist region, indicated generally at 14, and a crotch region, indicated generally at 15. The front and back waist regions 12, 14 have respective side regions 16, 18 which are attached to each other along side seams 19.

With particular reference to Figs. 3 and 4, the garment shell 22 comprises a front panel assembly, which is generally indicated at 26, having laterally opposite side margins 48 and a back panel assembly, which is generally indicated at 28 in Fig. 3, having laterally opposite side margins 50. The side margins 48, 50 of the front and back panel assemblies 26, 28 of the garment shell 22 are attached to each other to broadly define the side seams 19 of the absorbent garment 10, and to define the three-dimensional configuration of the garment shell during wear.

In its three-dimensional configuration as shown in Fig. 3, the garment shell 22 has a front waist region 32 which at least in part defines the front waist region 12 of the absorbent garment 10, a back waist region 34 which at least in part defines the back waist region 14 of the absorbent garment, and front and back waist ends, designated 56 and 58, respectively, which together generally define the waist opening 36 of the garment shell. In the illustrated embodiment, the garment shell 22 is configured to resemble a pair of shorts and thus further has a crotch region 38 extending longitudinally between and interconnecting the front waist region 32 and the back waist region 34 of the garment shell. The crotch region 38 of the garment shell 22 at least in part defines the crotch region 15 of the absorbent garment 10, and also in part defines leg openings 40 of the garment shell (broadly referred to herein as outer leg openings of the absorbent garment). However, it is understood that the crotch region 38 of the garment shell 22 may be omitted (so that the crotch region 15 of the absorbent garment 10 is defined solely by the absorbent assembly 24 as described later herein), such as where the garment shell is intended to resemble a skirt (in which case only one leg opening 40 of the garment shell is provided to accommodate both legs of the wearer), without departing from the scope of this invention.

Referring to Fig. 3, the front panel assembly 26 of the garment shell 22 comprises a pair of panel members 42 which are in particular embodiments permanently attached to each other, along a central seam 44 extending longitudinally from the front waist region 32 to the crotch region 38 of the garment shell 22. The back panel assembly 28 comprises a pair of panel members 46 configured and permanently attached to each other in a manner similar to the panel members 42 of the front panel assembly 26 along a central seam (not shown) extending longitudinally from the back waist region 34 to the crotch region 38 of the garment shell 22. It is understood, however, that each of the front and back panel assemblies 26, 28 may be constructed of a single panel member (e.g., of unitary construction) without departing from the scope of this invention. Alternatively, the front and back panel members 42, 46 on one side of the garment shell 22 may be formed integrally at the crotch region 38 thereof so that no attachment of the panel members is necessary at the leg openings.

The panel members 42, 46 of the front and back panel assemblies 26, 28 of the garment shell 22 can be constructed of any suitable disposable material, and more suitably a material that provides a generally cloth-like texture. The garment shell is desirably intended to be disposable after a single use. As an example, the panel members 42, 46 may be constructed from natural and/or synthetic sources and may be constructed in any suitable manner including, but not limited, to nonwovens such as spunbond-meltblown-spunbond film laminates, bonded carded web, spunlace, hydroentangled, and needle punched fabrics. The panel members 42, 46 are suitably liquid permeable, although it is understood that the panel members may be liquid impermeable without departing from the scope of this invention.

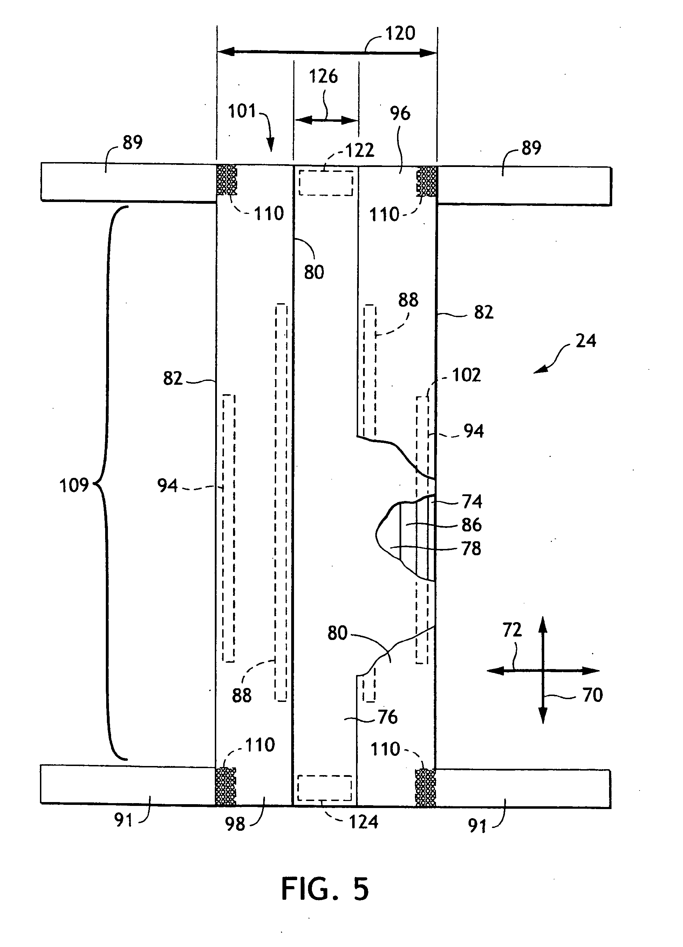

The absorbent assembly 24, as is illustrated in Fig. 5, is detached from the garment shell 22 and in a laid flat configuration. It is contemplated that the absorbent assembly 24 could be omitted from garment 10, leaving only the shell 22. In effect, this would create a disposable boxer short. The absorbent assembly 24 is illustrated as being rectangular in shape, and has a longitudinal axis 70 and a transverse, or lateral axis 72. It is understood that the absorbent assembly 24 may be other than rectangular, such as hourglass-shaped, T-shaped, I-shaped or other suitable shape without departing from the scope of this invention. Further, the absorbent assembly 24 may be configured like a jock-strap. The absorbent assembly 24 comprises an outer cover 74, a bodyside liner 76 in superposed relationship with the outer cover, an absorbent body 78 disposed between the outer cover and the bodyside liner, and a pair of laterally spaced containment flaps 80 configured to inhibit the transverse flow of body exudates on the liner to the side edges 82 of the absorbent assembly. The absorbent assembly 24 may further include a front belt member 89 and a back belt member 91.

Each belt member 89, 91 of absorbent garment 10 is used to enhance the fit of the absorbent assembly 24 within absorbent garment 10 by forming an inner waist band 101, see Figs. 3 and 4. More specifically, the belt members 89, 91, once attached to one another and to the absorbent assembly as described herein, are intended to provide tension along the waist ends 96, 98 of the absorbent assembly. It is further contemplated that the waist belts could be integrally connected to form a left belt member 105 and a right belt member 107 without departing from the intended scope of the invention. The belt members 89, 91 are suitably stretchable, and most desirably, suitably elastic.

The exterior cover 74 comprises a multi-layered laminate structure in which at least one of the layers is liquid impermeable. For instance, the exterior cover 74 can include a liquid permeable outer layer 84 and a liquid impermeable inner layer 86 which are suitably joined together by a laminate adhesive, ultrasonic bonds, pressure bonds, thermal bonds, or the like. The inner layer 86 of the exterior cover 74 can be both liquid and vapor impermeable, or it may be liquid impermeable and vapor permeable. Alternatively, the exterior cover 74 may comprise a single layer of liquid impermeable material.

The liquid permeable bodyside liner 76 is illustrated as overlying the exterior cover 74 and absorbent body 78, and may but need not have the same dimensions as the exterior cover 74. The bodyside liner 76 can be less hydrophilic than the absorbent body 78, to present a relatively dry surface to the wearer and to permit liquid to readily penetrate through the liner. Alternatively, the bodyside liner 76 can be more hydrophilic or can have essentially the same affinity for moisture as the absorbent body 78 to present a relatively wet surface to the wearer to increase the sensation of being wet. This wet sensation can be useful as a training aid. The hydrophilic/hydrophobic properties can be varied across the length, width and depth of the bodyside liner 76 and absorbent body 78 to achieve the desired wetness sensation or leakage performance.

The absorbent body 78 (Fig. 5) is positioned between the exterior cover 74 and the bodyside liner 76, which can be joined together by any suitable means.

Containment flaps 80 are located generally adjacent to the side edges 82 of the absorbent assembly 24, and can extend longitudinally along the entire length of the absorbent assembly 24 as shown in Fig. 5 or only partially along the length of the absorbent assembly. Flap elastic members 88 can be operatively joined with the containment flaps 80 in a suitable manner as is well known in the art. The elasticized containment flaps 80 can define a partially unattached distal edge (not shown), unattached to the liner 76, which assumes an upright configuration in at least the crotch region 90 of the absorbent assembly 24 during wear to form a seal (e.g., an elastic fit) against the wearer's body. It is understood, however, that the containment flaps 80 may be omitted without departing from the scope of this invention.

To enhance the fit of the absorbent garment 10 on the wearer and to further inhibit leakage of body exudates, the absorbent assembly may have leg elastic members 94 (Fig. 5), as arc known to those skilled in the art. The leg elastic members 94 can be operatively joined to the exterior cover 74 and/or the bodyside liner 76 and extend longitudinally adjacent the opposite side edges 82 generally through the crotch region 90 of the absorbent assembly 24.

To further enhance the fit of absorbent garment 10, absorbent assembly 24 it is operatively attached to the garment shell at the front and back waist regions 32 and 34.

The array of the present invention further includes a non-covered garment, such as a garment that appears more like a typical disposable diaper or disposable pant. In contrast to the shell-covered garment, when the non-covered garment is worn without any other clothing, it may be apparent that it is a disposable diaper or pant product. A general description of as non-covered garment follows.

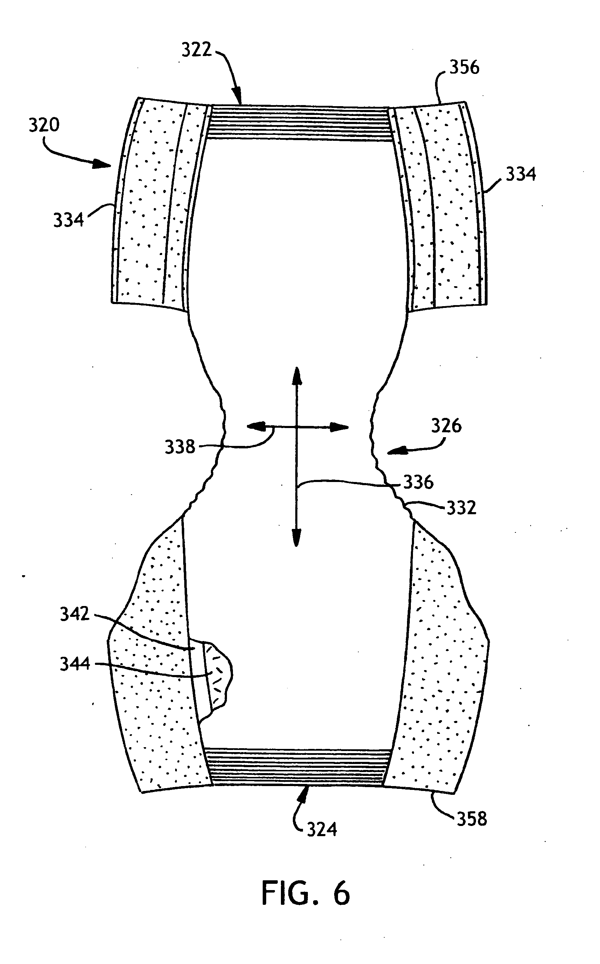

Referring now to the Figs. 6 and 7, a non-covered garment or pant 320 is illustrated in a fully assembled condition and in a partially disassembled, stretched and laid flat condition. The non-covered garment does not include a shell 22. The pant 320 defines a first or front waist region 322, a second or back waist region 324, a crotch region 326 positioned between and interconnecting the front and back waist regions, an inner surface 328 (Fig. 7) which is configured to contact the wearer, and an outer surface 330 opposite the inner surface which is configured to contact the wearer's clothing. The illustrated pant 320 comprises an absorbent chassis 332 and a plurality of transversely opposed side panels 334. The absorbent chassis 332 and side panels 334 can be integrally formed or comprise two or more separate elements, as shown.

The pant 320 defines a longitudinal centerline 336, a transverse centerline 338, a first or front longitudinal end edge 356, and a second or back longitudinal end edge 358. The first waist region 322 abuts the first longitudinal end edge 356, and the second waist region 324 abuts the second longitudinal end edge 358. "Longitudinal" and "transverse" have their customary meaning. The longitudinal axis lies in the plane of the article and is generally parallel to a vertical plane that bisects a standing wearer into left and right body halves when the article is worn. The transverse axis lies in the plane of the article generally perpendicular to the longitudinal axis. The article as illustrated is longer in the longitudinal direction than in the transverse direction.

The illustrated absorbent chassis 332 comprises an outer cover 340 and a bodyside liner 342 which is connected to the outer cover in a superposed relation. The absorbent chassis 332 also comprises an absorbent assembly 344 which is located between the outer cover 340 and the bodyside liner 342, and can optionally include a pair of containment flaps (not shown).

With the pant 320 in a fully assembled condition as illustrated in Figs. 6 and 7, the front and back waist regions 322 and 324 are joined together by side seams 346 to define a waist opening 350 and a pair of leg openings 352. The front waist region 322 comprises the portion of the pant 320 which, when worn, is positioned on the front of the wearer while the back waist region 324 comprises the portion of the training pant which, when worn, is positioned on the back of the wearer. The crotch region 326 of the pant 320 comprises the portion of the training pant which, when worn, is positioned between the legs of the wearer and covers the lower torso of the wearer. The side panels 334 comprise the portions of the pant 320 which, when worn, are positioned on the side hip regions of the wearer. The longitudinal end edges 356 and 358 of the pant 320 are configured to encircle the waist of the wearer when worn and provide the waist opening 350.

The absorbent chassis 332 is configured to contain and/or absorb any body exudates discharged from the wearer. For example, the absorbent chassis 332 desirably although not necessarily comprises the pair of containment flaps (not shown) which can be configured to provide a barrier to the transverse flow of body exudates. Suitable constructions and arrangements for the containment flaps are generally well known to those skilled in the art and are described in U.S. Pat. No. 4,704,116 issued Nov. 3, 1987 to Enloe.

To further enhance containment and/or absorption of body exudates, the pant 320 can include a front waist elastic member, a rear waist elastic member, and leg elastic members (not shown), as are known to those skilled in the art. Waist elastic members and leg elastic members can be operatively joined to the outer cover 340 and/or bodyside liner 342 of the pant 320. Elastic members for the containment flaps, waist elastics and leg elastics can be formed of any suitable elastic material. As is well known to those skilled in the art, suitable elastic materials include sheets, strands or ribbons of natural rubber, synthetic rubber, or thermoplastic elastomeric polymers.

The outer cover 340 has an exterior surface corresponding to the outer surface 330 of the training pant and an opposite interior surface (not shown). The outer cover 340 preferably comprises a material which is substantially liquid impermeable. The outer cover 340 can be a single layer of liquid impermeable material, but preferably comprises a multi-layer laminate structure in which at least one of the layers is liquid impermeable.

For additional detail regarding the construction of the pant, including the cover 340, the bodyside liner 342 and the absorbent material 344, reference may be made to the following patents/publications assigned to Kimberly-Clark Worldwide, Inc., incorporated herein by reference to the extent they are consistent with the present invention: US Patent Nos. 4,940,464; 4,938,757; 6,613,033; and Patent Publication Nos. US20040127878; US20040102755; US20050085784; US20050256489; and US20060004341.

When the outward appearance of shell-covered garment 10 is compared to the outward appearance of the non-covered garment 320, the very existence of the shell 22 will cause a difference in the type of discretion. An absorbent article that provides alternate discretion may fit more loosely, and/or cover a greater percentage of body surface area. Further, the shell 22 may have varying degrees of opacity.

One technique of determining whether or not an absorbent product fits loosely is a comparison of product dimensions to wearer dimensions at a particular body location, such as at the wearer's hip. Absorbent garments are typically manufactured and marketed to fit a range of wearer sizes/weights, as expressed on package graphics and information. Data are publicly available that document ranges of dimensions and other properties of the human body, called anthropometric data. Comparison of absorbent garment dimensions to anthropometric data for bodies in the weight range targeted by the absorbent garment can provide an indication of whether or not the garment is intended to fit the body loosely.

Data from an anthropometric study of hip circumference and weight is shown in Tables 1 and 2, respectively. (Table data source: Sandy Ressler, AnthroKids - Anthropometric Data of Children, United States National Institute of Standard and Technology, 1977). The hip circumference is defined as the maximum horizontal circumference of the hips at the level of the greatest posterior protrusion of the buttocks, as viewed from the side of an individual standing erect.

**Table 1.**

| **HIP CIRCUMFERENCE CM. - (MALES AND FEMALES)** | | | | | | | | |
|---|---|---|---|---|---|---|---|---|
| **AGE/YRS** | **N** | **MEAN** | **S.D.** | **MIN** | **5TH** | **50TH** | **95TH** | **MAX** |
| 2.0-3.5 | 200 | 51.7 | 3.2 | 43.8 | 46.9 | 51.8 | 57.0 | 62.1 |
| 3.5-4.5 | 225 | 53.5 | 3.5 | 45.4 | 48.2 | 53.1 | 59.4 | 65.6 |
| 4.5-5.5 | 260 | 56.1 | 3.5 | 48.2 | 51.2 | 55.6 | 62.2 | 77.7 |
| 5.5-6.5 | 217 | 58.3 | 4.0 | 49.8 | 52.0 | 58.0 | 65.7 | 70.0 |
| 6.5-7.5 | 222 | 61.9 | 5.2 | 49.1 | 55.2 | 60.9 | 71.7 | 84.6 |
| 7.5-8.5 | 188 | 65.0 | 5.6 | 55.9 | 57.7 | 64.0 | 76.2 | 90.8 |
| 8.5-9.5 | 248 | 67.7 | 5.9 | 55.3 | 59.4 | 67.0 | 79.8 | 91.4 |
| 9.5-10.5 | 247 | 70.8 | 6.7 | 59.4 | 61.7 | 69.8 | 82.8 | 108.5 |
| 10.5-11.5 | 276 | 74.3 | 7.7 | 59.3 | 64.2 | 73.1 | 90.6 | 108.2 |
| 11.5-12.5 | 283 | 76.6 | 7.2 | 62.3 | 66.2 | 75.7 | 90.1 | 101.9 |
| 12.5-13.5 | 310 | 81.0 | 7.3 | 64.8 | 70.0 | 79.8 | 93.3 | 107.3 |
| 13.5-14.5 | 270 | 84.3 | 7.1 | 60.2 | 72.9 | 84.3 | 96.5 | 104.2 |
| 14.5-15.5 | 262 | 88.1 | 7.1 | 69.5 | 75.6 | 88.2 | 99.0 | 115.9 |
| 15.5-16.5 | 198 | 90.5 | 6.3 | 67.1 | 80.9 | 90.1 | 101.5 | 108.9 |
| 16.5-17.5 | 220 | 91.8 | 6.4 | 78.7 | 82.9 | 91.0 | 104.6 | 116.0 |
| 17.5-19.0 | 156 | 93.0 | 6.0 | 77.1 | 84.2 | 92.4 | 103.4 | 112.5 |

**Table 2.**

| **WEIGHT KG. - (MALES AND FEMALES)** | | | | | | | | |
|---|---|---|---|---|---|---|---|---|
| **AGE/YRS** | **N** | **MEAN** | **S.D.** | **MIN** | **5TH** | **50TH** | **95TH** | **MAX** |
| 2.0-3.5 | 212 | 14.1 | 1.9 | 10.1 | 11.3 | 14.0 | 17.1 | 20.7 |
| 3.5-4.5 | 228 | 16.2 | 2.1 | 11.4 | 13.1 | 16.0 | 19.6 | 23.8 |
| 4.5-5.5 | 271 | 18.3 | 2.5 | 13.7 | 15.1 | 17.9 | 22.1 | 36.0 |
| 5.5-6.5 | 243 | 20.5 | 3.0 | 13.1 | 16.1 | 20.0 | 25.6 | 32.5 |
| 6.5-7.5 | 231 | 23.7 | 4.1 | 15.4 | 18.3 | 22.9 | 31.1 | 43.6 |
| 7.5-8.5 | 197 | 26.6 | 5.2 | 17.6 | 20.4 | 25.5 | 36.9 | 54.2 |
| 8.5-9.5 | 256 | 29.7 | 5.5 | 19.6 | 22.7 | 28.6 | 40.0 | 60.0 |
| 9.5-10.5 | 258 | 33.1 | 6.6 | 22.2 | 24.8 | 31.9 | 45.5 | 69.0 |
| 10.5-11.5 | 282 | 37.2 | 8.2 | 20.7 | 27.4 | 35.2 | 53.2 | 80.0 |
| 11.5-12.5 | 287 | 40.3 | 8.1 | 26.1 | 29.3 | 39.1 | 54.7 | 77.8 |
| 12.5-13.5 | 315 | 45.7 | 9.0 | 26.5 | 33.6 | 44.2 | 62.0 | 85.8 |
| 13.5-14.5 | 271 | 50.8 | 9.2 | 29.8 | 37.7 | 50.0 | 67.8 | 77.3 |
| 14.5-15.5 | 264 | 55.5 | 10.0 | 32.7 | 40.2 | 54.2 | 72.1 | 105.0 |
| 15.5-16.5 | 198 | 60.7 | 11.5 | 33.0 | 44.7 | 58.9 | 80.1 | 106.6 |
| 16.5-17.5 | 221 | 62.2 | 11.6 | 36.4 | 47.0 | 60.1 | 81.1 | 106.2 |
| 17.5-19.0 | 156 | 65.7 | 13.1 | 41.4 | 45.6 | 65.4 | 86.5 | 112.3 |

A "loose fit" will require that the effective hip measurement of a garment be larger than the mean hip measurement of a wearer of a predetermined weight. The corresponding hip size and weight can be determined by the age ranges listed in Tables 1 and 2. For example, a person having a mean weight of 45.7 kg has a mean hip circumference of 81 cm. The effective hip measurement of a garment is determined by the test method provided herein.

With respect to opacity of a garment shell 22, it is desirable that the opacity be at least 50 percent. In another embodiment of the invention, it is desirable that the opacity be at least 60 percent. In a further embodiment of the invention, it is desirable that the opacity be at least 70 percent. In yet another embodiment of the invention, it is desirable that the opacity be at least 80 percent. The higher the opacity of shell 22, the more discreet the garment 10 is because there is less ability for others to see the absorbent member through the shell when garment 10 is worn.

With respect to the coverage area for any garment in the array, such as garment 10 or 320, it is desirable that shell-covered garment 10 cover a larger surface area of the wearer than the non-covered garment, as determined by the test method herein. Each garment compared by the test method is configured to fit a wearer in a particular size range, or having about the same mean hip circumference. In one embodiment, the shell-covered garment 10 may cover at least 10 percent more surface area than the non-covered garment 320. In another embodiment, the shell-covered garment 10 may cover at least 15 percent more surface area than the non-covered garment 320. In a further embodiment, the shell-covered garment 10 may cover at least 20 percent more surface area than the non-covered garment 320.

In yet another embodiment, the shell-covered garment 10 may cover at least 25 percent more surface area than the non-covered garment 320.

### TEST METHODS

### ISO Opacity Test Method

The Opacity of the test materials may be tested using a Technibrite Micro TB-1C apparatus, available from Technidyne Corporation, New Albany, Indiana, U.S.A.

Test material samples consist of garment portions such as a portion of shell 22. The samples should be clean and free of defects, and the testing areas on the samples to be analyzed should be free from perforation lines. If the shell 22 is not of a solid color, then the garment must be divided into as many test material samples as possible. An attempt must be made to include all indicia or color value gradations disposed on the outer surface if the garment.

The samples are cut into square shapes approximately 2.25 inches (5.7 cm) on each side. If the specimens are of a solid color, at least 10 specimens should be tested individually. If the specimens are not of a solid color then at least 50 specimens should be tested individually. Stacks of specimens should be used as "full opacity" controls; the number of specimens per stack should be chosen so that no noticeable change in readings results when additional sheets are added. Each stack should be used as a control for each of five different individual specimens; therefore, for 10 individual specimens, two "full opacity" control stacks will be required.

The tests should be conducted in a standard laboratory atmosphere of 23 ± 1 °C (73.4 ± 1.8°F) and 50 ± 2% relative humidity. A black body cup must be on the sample holder for all single-sheet opacity readings. A Y (green) filter should be in the active position. The infinite stacks and individual specimens should remain in order; i.e., stack 1 with specimens 1-5; stack 2 with specimens 6-10; etc.

The instrument is turned on and allowed to warm up for at least 30 minutes before testing any samples. The instrument is calibrated as directed in the instrument manufacturer's instruction manual. The calibration is verified at least once per day, prior to testing. The swing-in standard should be kept clean and free of fingerprints, using lint-free wipes or lens paper and a lens cleaner that does not leave a residue, as needed.

Data obtained are expressed as percent opacity, where the "full opacity" control stack represents 100%. The opacity value for a material should be calculated by averaging the percent opacity values for at least 10 individual specimens of that material.

The following procedure should be used:
1. Press the REPROGRAM button and select ISO OPACITY for data output.
2. Press PRINT to complete the routine and check the selected output type for correctness.
3. Press the QC button, enter the sample number, and press PRINT again.
4. Load stack 1 onto the spring-loaded sample holder. Release the spring-loaded sample holder, with the stack, so it is tight against the port.
5. Press SCAN.
6. Remove the stack.
7. Place individual sample 1 onto the spring-loaded sample holder. Press PRINT.
8. Repeat steps 4-7 with successive specimens until individual specimens 1-5 have each been tested.
9. Repeat steps 2-8 for stack 2 and specimens 6-10; these steps can be repeated as many times as needed until all individual specimens have been tested against their respective stacks. Keep individual samples and sets in order.
10. Average the individual "Calculated TAPPI opacity" values from the full set of tests.

### Garment Looseness Test Method

The garments 10 and 320 described above should be analyzed as follows: The garment of the array (e.g. garment 10 or 320) should be removed from the package and gently spread flat with front side up (i.e., with legs or leg openings side by side, rather than overlapping), without stretching. The garment of the array should be marked at a location 6 inches (15 cm) below the top waist edge of garment, at least 10 locations approximately evenly spaced (such as 5 on the garment of the array front, 5 on the garment back). If the garment does not contain a shell 22, the garment should be gently spread flat, front side up, and extended without stretching, to provide a generally linear array of marking points. The width of the flattened garment should be measured along the line of marking points, and multiplied by two. This number serves as the effective hip circumference of the garment.

If the garment contains a shell 22, the shell 22 only is marked as described. If the shell 22 is gathered, elasticized or otherwise non-flat in the relaxed garment, such as may result from an elastic waist region, the shell 22 is separated from the elasticized waistband or other gathered area such as by cutting. Separation is done without stretching or otherwise deforming the material of the shell. The shell 22 should then be gently spread flat and extended as described above to provide a generally linear array of marking points. The width of the flattened shell 22 is measured along the line of marking points, and multiplied by two. This number serves as the effective hip circumference of the garment.

The effective hip circumference of the garment is compared to the mean hip circumference of wearer at the mid-point of the garment's weight range (male and female combined, for garments intended for use by either gender, or that of a specific gender when the garment is intended for use by only one gender). Anthropometric data such as that seen in Table 1 may be used for a given age range. It is desirable to update the anthropometric data as such data tend to change over time. A garment with effective hip circumference equal to or less than the mean hip circumference of wearers at the mid-point of the garment's intended weight range is classified as a close-fitting garment. A garment with effective hip circumference greater than the mean hip circumference of wearers at the mid-point of the garment's intended weight range is classified as a loose-fitting garment. A first garment that has an effective hip circumference that is larger than a second garment effective hip circumference is more loose-fitting than the second garment.

### Surface Coverage Test Method

The extent of coverage of body surface area provided by two different garments may be assessed visually, such as by examination of photographs of a mannequin wearing both garments. One such approach involves taking photographs of a mannequin wearing each garment. Photographs are taken from the same perspective (i.e. relative vertical placement), at the same distance from the mannequin, and the mannequin is disposed in two positions relative to the camera (front facing and rear facing). For each garment, there is one picture taken with the mannequin unclothed, and several other pictures taken with the mannequin wearing a garment specimen. A sample of four identical garment-types having identical sizes are used for a total of 10 photographs (four front and rear views with garments, one front and rear view without garments). The pictures are analyzed by determining the mean surface area of the mannequin covered by the garment after combining results from both the front and the rear views.

As various changes could be made in the above constructions without departing from the scope of the invention, it is intended that all matter contained in the above description or shown in the accompanying drawings shall be interpreted as illustrative and not in a limiting sense.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements. "Disposed," "disposed on," "disposed with," "disposed at," "disposed near" and variations thereof are intended to mean that one element can be integral with another element, or that one element can be a separate structure bonded to or placed with or placed near another element.

## Claims

1. An array of disposable absorbent articles, wherein the articles are worn about the lower torso and are adapted for a user in a particular life stage, the array being manufactured by or for the same business entity and comprising:
a non-covered absorbent garment (320);
**characterized in that** the array further comprises:
a first shell-covered absorbent garment (10) comprising a first shell (22) and a first absorbent member (24);
a second shell-covered garment comprising a second shell and a second absorbent member; and
wherein the first shell-covered garment comprises a first shell having a configuration selected from the group consisting of a boxer short, a skirt, bloomers, boxer briefs, a skort, and trousers; and the second shell-covered garment comprises a second shell having a different configuration selected from the group consisting of a boxer short, a skirt, bloomers, boxer briefs, a skort, and trousers.

2. The array according to claim 1 wherein the first shell-covered garment comprises a different type of discretion than the non-covered garment with respect to one or more of the following characteristics selected from the group consisting of opacity, coverage area, and looseness of fit.

3. The array according to claim 2 wherein the first shell-covered garment has greater coverage than the second shell-covered garment in accordance with the Surface Coverage Test Method; or wherein the first shell-covered garment has a loose fit in accordance with the Garment Looseness Test Method.

4. The array according to any preceding claim wherein the first shell and the second shell comprise a nonwoven material.

5. The array of any preceding claim wherein the articles are pant-like garments for enuresis or incontinence needs.

## Patentansprüche

1. Anordnung aus absorbierenden Einwegartikeln, wobei die Artikel am Unterkörper getragen werden und für einen Benutzer in einem bestimmten Lebensstadium eingerichtet sind, wobei die Anordnung durch oder für ein und dasselbe Unternehmen hergestellt ist und Folgendes umfasst:
ein nicht abgedecktes, absorbierendes Kleidungsstück (320),
**dadurch gekennzeichnet, dass** die Anordnung ferner Folgendes umfasst:
ein erstes hüllen-abgedecktes, absorbierendes Kleidungsstück (10), das eine erste Hülle (22) und ein erstes absorbierendes Element (24) umfasst,
ein zweites hüllen-abgedecktes Kleidungsstück, das eine zweite Hülle und ein zweites absorbierendes Element umfasst, und
wobei das erste hüllen-abgedeckte Kleidungsstück eine erste Hülle umfasst, die eine Gestaltung aufweist, die aus der Gruppe ausgewählt ist, die aus Boxershorts, einem Rock, Damenunterhosen, Retroshorts, einem Skort und Hosen besteht, und das zweite hüllen-abgedeckte Kleidungsstück eine zweite Hülle umfasst, die eine andere Gestaltung aufweist, die aus der Gruppe ausgewählt ist, die aus Boxershorts, einem Rock, Damenunterhosen, Retroshorts, einem Skort und Hosen besteht.

2. Anordnung nach Anspruch 1, wobei das erste hüllen-abgedeckte Kleidungsstück in Hinsicht auf eine oder mehrere der folgenden Eigenschaften, die aus der Gruppe ausgewählt sind, die aus Blickdichte, Bedeckungsbereich und Lockerheit der Passform besteht, eine andere Art von Sichtschutz als das nicht abgedeckte Kleidungsstück umfasst.

3. Anordnung nach Anspruch 2, wobei das erste hüllen-abgedeckte Kleidungsstück gemäß dem Oberflächen-bedeckungs-Prüfver-fahren einen größeren Bedeckungsbereich aufweist als das zweite hüllen-abgedeckte Kleidungsstück oder wobei das erste hüllen-abgedeckte Kleidungsstück gemäß dem Bekleidungslockerheits-Prüfverfahren eine lockere Passform aufweist.

4. Anordnung nach einem der vorhergehenden Ansprüche, wobei die erste Hülle und die zweite Hülle ein Vliesmaterial umfassen.

5. Anordnung nach einem der vorhergehenden Ansprüche, wobei die Artikel hosenartige Kleidungsstücke für den Gebrauch bei Bettnässen oder Inkontinenz sind.

## Revendications

1. Ensemble d'articles absorbants jetables, dans lequel les articles sont portés autour du torse inférieur et adaptés à un utilisateur dans un stade de vie particulier, l'ensemble étant fabriqué par ou pour la même entité commerciale et comprenant :
un vêtement absorbant non couvert (320) ;
**caractérisé en ce que** l'ensemble comprend en outre :
un premier vêtement absorbant (10) couvert d'une garniture, comprenant une première garniture (22) et un premier élément absorbant (24) ;
un second vêtement couvert d'une garniture, comprenant une seconde garniture et un second élément absorbant ; et
dans lequel le premier vêtement couvert d'une garniture comprend une première garniture ayant une configuration choisie dans le groupe constitué d'un caleçon, d'une jupe, d'une culotte bouffante, d'un slip, d'une jupe-short et d'un pantalon ; et le second vêtement couvert d'une garniture comprend une seconde garniture ayant une configuration différente choisie dans le groupe constitué d'un caleçon, d'une jupe, d'une culotte bouffante, d'un slip, d'une jupe-short et d'un pantalon.

2. Ensemble selon la revendication 1, dans lequel le premier vêtement couvert d'une garniture comprend un type de discrétion différent de celui du vêtement non couvert par rapport à une ou plusieurs des caractéristiques suivantes choisies dans le groupe constitué de l'opacité, de la surface couvrante et de l'ampleur d'ajustement.

3. Ensemble selon la revendication 2, dans lequel le premier vêtement couvert d'une garniture a une plus grande surface couvrante que le second vêtement couvert d'une garniture selon le procédé d'essai de surface couvrante ; ou dans lequel le premier vêtement couvert d'une garniture a un ajustement ample selon le procédé d'essai d'ampleur de vêtement.

4. Ensemble selon l'une quelconque des revendications précédentes, dans lequel la première garniture et le seconde garniture comprennent un matériau non tissé.

5. Ensemble selon l'une quelconque des revendications précédentes, dans lequel les articles sont des vêtements de type slip pour répondre à des besoins d'énurésie ou d'incontinence.
